**Europäisches Patentamt**

**European Patent Office**

**Office européen des brevets**

(19)

(11) Veröffentlichungsnummer: **0 156 257**
**A2**

## (12) EUROPÄISCHE PATENTANMELDUNG

(21) Anmeldenummer: **85102952.0**

(22) Anmeldetag: **14.03.85**

(51) Int. Cl.⁴: **A 61 F 13/18**

(30) Priorität: **28.03.84 DE 3411457**

(43) Veröffentlichungstag der Anmeldung:
**02.10.85 Patentblatt 85/40**

(84) Benannte Vertragsstaaten:
**AT BE CH IT LI LU NL**

(71) Anmelder: **Vereinigte Papierwerke Schickedanz & Co.**
**Schoppershofstrasse 80**
**D-8500 Nürnberg(DE)**

(72) Erfinder: **Haegler, Walter, Dr.**
**Holbeinstrasse 5**
**D-8507 Oberasbach(DE)**

(72) Erfinder: **Petranyi, Pal, Dr.**
**Schweriner Strasse 15**
**D-8500 Nürnberg(DE)**

(72) Erfinder: **Hübner, Peter**
**Waldstrasse 7**
**D-8501 Winkelhaid(DE)**

(74) Vertreter: **Pohl, Hans Ludwig (DE)**
**Hefnersplatz 3**
**D-8500 Nürnberg 11(DE)**

(54) **Damenbinde oder Slipeinlage.**

(57) Es wird ein hygienischer Zellstoffartikel, beispielsweise eine Damenbinde, beschrieben, der einen Saugkörper, eine poröse Vliesstoffabdeckung und eine Wäscheschutzfolie aufweist. Der Saugkörper ist wenigstens mit seiner unteren Schicht mit der Wäscheschutzfolie verklebt. Die Wäscheschutzfolie oder der Zellstoffartikel weist auf der Unterseite eine Haftschmelzkleberschicht auf. Die den Saugkörper mit der Wäscheschutzfolie verbindende Klebstoffschicht besteht aus einem solchen Heiss-Schmelzklebstoff oder Dispersionsklebstoff, der den Zellstoffartikel in Quer- und Längsrichtung versteift.

Vereinigte Papierwerke
Schickedanz & Co.

Schoppershof
8500 Nürnberg

0156257

HP/5/ - B 71 D
26.03.1984

I

- 4 -

## Damenbinde oder Slipeinlage

Die Erfindung betrifft hygienische Zellstoffartikel wie
Damenbinden oder Slipeinlagen.

Hygienische Zellstoffartikel dieser Art sind in verschiedenen Ausführungsformen bekannt. Sie bestehen allgemein
aus einem Saugkörper, einer porösen Vliestoffabdeckung und
einer Wäscheschutzfolie. Bei manchen Ausführungsformen,
insbesondere bei Slipeinlagen und sogen. dünnen Binden,
ist der Saugkörper wenigstens mit seiner unteren Schicht
mit der Wäscheschutzfolie verklebt und auf diese Weise
gegen Querverschiebung gesichert. Die Wäscheschutzfolie
oder auch der Zellstoffartikel weist dabei häufig auf der
Unterseite eine Haftschmelzkleberschicht auf, mit der der
Zellstoffartikel an oder in einem Wäschestück befestigt
werden kann. Diese Haftschmelzkleberschicht ist vor Gebrauch meist mit einer Deckfolie, beispielweise einem paraffinierten oder silikonisierten Papier abgedeckt, welches vor Gebrauch leicht abgenommen werden kann. Hygienische Zellstoffartikel dieser Art sind beispielsweise in
der deutschen Offenlegungsschrift 21 07 426 beschrieben.

Beim Gebrauch derartiger Artikel hat sich deren Tendenz
als nachteilig herausgestellt, sich beim Tragen in Querrichtung, manchmal auch in Längsrichtung oder in beiden
Richtungen zusammenzufalten oder -zurollen. Die Erscheinung ist nachteilig, da sie zwangsläufig dazu führt, den
Artikel gegen einen frischen auszutauschen, auch wenn dies
mit Rücksicht auf die Verschmutzung an sich noch nicht
erforderlich wäre.

- 5 -

Vereinigte Papierwerke
Schickedanz & Co.

Schoppershofstr. **0156257**
8500 Nürnberg

HP/5/ - B 71 D
26.03.1984

2
- 5 -

Der Erfindung liegt die Aufgabe zugrunde, die vorbekannten hygienischen Zellstoffartikel der beschriebenen Art dahingehend abzuwandeln, daß sie eine höhere Stabilität gegen Zusammenfalten und -rollen aufweisen. Zur Lösung dieser Aufgabe wird vorgeschlagen, daß die den Saugkörper mit der Wäscheschutzfolie verbindende Klebstoffschicht aus einem Heißschmelzklebstoff oder Dispersionsklebstoff besteht, der den Zellstoffartikel in Quer- und Längsrichtung versteift.

Es hat sich gezeigt, daß manche Klebstoffarten hierzu besonders geeignet sind, insbesondere solche Klebstoffarten, die auf Basis kurzkettiger Paraffinwachse mit hoher Kristallinität aufgebaut sind und die weitere Anteile von mikrokristallinen Wachsen und/oder Elastomere auf der Basis Polyvinylacetat, Polyvinylpropionat, Ethylen-Vinylacetat-Copolymere, Polyacrylsäureester, Butadien-Styrol-Copolymere und Blockcopolymere auf Basis Styrol-Isopren-Styrol oder Styrol-Butadien-Styrol, sowie klebrigmachende Harze auf Basis Kolophonium und seiner Derivate, Kohlenwasserstoffharze mit aliphatischem, aromatischem oder alkylaromatischem Charakter enthalten. Wird die an sich bekannte Klebeverbindung zwischen der Unterseite des Saugkörpers sowie der Wäscheschutzfolie unter Verwendung solcher Klebstoffschichten hergestellt, so tritt die gewünschte Quer- und Längsversteifung ein und es entsteht eine besser formstabile Binde oder Einlage, die dennoch allen gewohnten Tragekomfort, insbesondere hinsichtlich Weichheit, aufweist.

Als vorteilhaft hat es sich erwiesen, wenn die Klebstoffschicht ein Flächengewicht von 30 - 200 g/qm, vorzugsweise
100 bis 150 g/qm aufweist. Die Klebstoffschicht kann dabei
vollflächig oder in beliebigen Mustern ausgeführt sein.
Als Muster kommen z. B. Querstreifen, Rauten, Ellipsen
oder Kreise in Betracht; welche Anordnung und welches genaue Flächengewicht im einzelnen Fall optimal ist, muß
jeweils in Abhängigkeit von der Klebstoffzusammensetzung
durch Tastversuche ermittelt werden.

Als vorteilhaft hat es sich weiter erwiesen, wenn die
Klebstoffschicht wenigstens 2 mm, besser 5 bis 10 mm
schmäler ist, als die Breite des Saugkörpers. Die Länge
der Klebstoffschicht sollte das 0,5- bis 0,75-fache der
Länge des Saugkörpers betragen und im Mittelbereich angeordnet sein.

Desweiteren wurde festgestellt, daß der beschriebene Versteifungseffekt noch vergrößert werden kann, wenn die Erfindung auf solche Zellstoffartikel angewandt wird, bei
denen die poröse Vliesstoffabdeckung als Umhüllung ausgeführt ist, deren Ränder sich an der Unterseite des Zellstoffartikels überlappen und dort miteinander verklebt
sind. Bei diesen Zellstoffartikeln bildet die Unterseite
der Wäscheschutzfolie also nicht auch die Unterseite des
Zellstoffartikels, sondern der Artikel wird noch von den
Rändern der porösen Vliesstoffabdeckung überdeckt, was dem
Gesamtartikel ein besseres taktiles Gepräge verleiht. Für
solche Artikel wird nun vorgeschlagen, daß die ohnehin an
der Unterseite erforderliche Haftschmelzkleberschicht, die
ja zum Festlegen des Artikels im Wäschestück benötigt

- 9 -

Vereinigte Papierwerke
Schickedanz & Co.

Schoppersh. 0156257
8500 Nürnberg

HP/5/ - B 71 D
26.03.1984

- 7 -

wird, die Vliesstoffumhüllung im Bereich der Unterseite wenigstens teilweise durchdringt und die Umhüllung mit der Unterseite der Wäscheschutzfolie verbindet. Durch diese Maßnahme wird erreicht, daß auch die Umhüllung gegen Querverschiebung stabilisiert ist, und daß gleichzeitig ein mehrschichtiger Stabilisierungsblock im Inneren des Zellstoffartikels entsteht, der den gewünschten Versteifungseffekt vervielfältigt. Der Stabilisierungsblock besteht somit aus drei Schichten, nämlich der oberen Klebstoffschicht mit daran gebundenem Saugkörper, der Wäscheschutzfolie, und der unteren Haftklebstoffschicht, die den einhüllenden Vliesstoff durchdringt.

Naturgemäß ist es nicht erforderlich, daß die Vliesstoffumhüllung im Bereich der Unterseite vollständig vom Haftschmelzkleber durchdrungen ist. Es genügt vielmehr bereits, wenn die Vliesstoffumhüllung in den Bereichen penetriert wird, in denen sich die Vliesstoffumhüllung nicht überlappt. Andererseits ist es natürlich besonders vorteilhaft, die Penetration des Haftschmelzklebers auch (oder vielleicht auch ausschließlich) im Überlappungsbereich der Vliesstoffumhüllung durchzuführen, da auf diese Weise die separat einzubringende Verklebung der sich überlappenden Teile der Vliesstoffumhüllung eingespart wird.

Der Erfindungsgegenstand wird im folgenden unter Bezugnahme auf die beigefügte Zeichnung näher erläutert. In der Zeichnung ist als Ausführungsbeispiel ein Teil einer Damenbinde perspektivisch in stark vergrößerter und teilweise aufgeschnittener Form gezeigt.

- 8 -

Vereinigte Papierwerke
Schickedanz & Co.

Schoppershof 0156257
8500 Nürnberg

HP/5/ - B 71 D
26.03.1984

- 8 -

Die dargestellte Damenbinde ist als Ganzes mit 1 bezeichnet. Die Darstellung ist so gewählt, daß sich die Unterseite 2 oben und die Gebrauchsseite 3 unten befindet.

Die dargestellte Damenbinde weist einen Saugkörper 4 auf, der in bekannter Weise aus Zellstofflocken gegebenenfalls in Mischung mit Quellstoffen besteht. Der Saugkörper 4 ist an der Gebrauchsseite mit einer porösen Vliesstoffschicht 5 abgedeckt, welche um die Ränder 6 herumgefaltet ist und auch die Unterseite 2 umgreift. Die Vliesstoffschicht 5 überlappt sich an der Unterseite 2, und die sich überlappenden Teile 7; 7' sind mit einer Klebstoffnaht 8 miteinander verklebt.

Die Unterseite des Saugkörpers ist desgleichen noch mit einer Wäscheschutzfolie 9 umgeben, deren Ränder 10 sich bis in den Randbereich 4' des Saugkörpers 4 erstrecken und diesen folglich von unten ähnlich wie eine U-förmige Rinne umgeben. Die Wäscheschutzfolie 9 ist, wie die Zeichnung zeigt, zwischen dem Saugkörper 4 und der Unterseite der Vliesstoffschicht 5 angeordnet.

An der Unterseite der Damenbinde befindet sich desweiteren noch eine Haftschmelzkleberschicht 11, die der Befestigung der Slipeinlage an oder in einem Wäschestück dient und die normalerweise bei Nichtgebrauch mit einem paraffinierten oder silikonisierten Papierstreifen abgedeckt ist, der aber in der Zeichnung nicht dargestellt ist.

- 9 -

- 8 -

Bei der dargestellten Damenbinde ist der Saugkörper 4 mit der Wäscheschutzfolie 9 durch eine Klebstoffschicht 12 verbunden, welche aus einem Heißschmelzklebstoff oder einem Dispersionsklebstoff besteht und die die Damenbinde in Quer- und Längsrichtung versteift. Zusätzlich kann auch die Haftschmelzkleberschicht 11 die Unterseite der Vliesstoffschicht 5 durchdringen (11') und diese somit mit der Unterseite der Wäscheschutzfolie verkleben. Es entsteht in diesem Fall ein Stabilierungsblock, bestehend aus der Klebstoffschicht 12, der Wäscheschutzfolie 9 sowie der Vliesstoffschicht 5, welche durchdrungen ist von der Haftschmelzkleberschicht 11 und 11'.

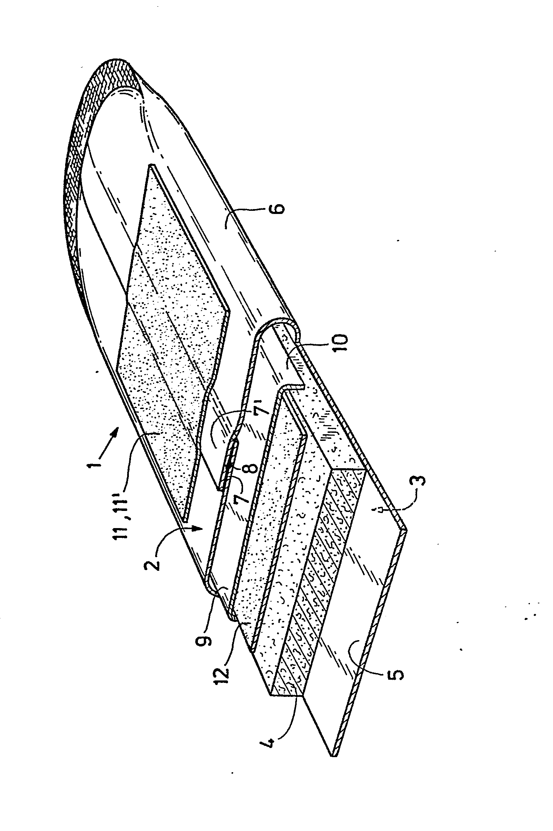

Bei der Herstellung eines derartigen hygienischen Zellstoffartikels kann ähnlich vorgegangen werden, wie dies in der deutschen Offenlegungsschrift 21 07 426, dort insbesondere Figur 3, dargestellt ist. Anstelle des dort vorgeschlagenen Klebstoffes (es handelt sich um Dextrinlösung) muß hier natürlich der erwähnte Heißschmelzklebstoff verwendet werden. Vorteilhafterweise wird eine Sorte eingesetzt, die infolge ihrer Zusammensetzung einen Erweichungspunkt (z. B. nach Ring- und Kugelmethode ASTM E 28 gemessen) von unter 90 °C aufweist. Die Applikationstemperatur muß je nach verwendetem Folienrohstoff unter 120 °C bzw. unter 150 °C liegen. Die Zusammensetzung muß so gewählt sein, daß der entstehende Hot-melt-Film im Temperaturbereich von 15 °C bis ca. 37 °C steif, aber elastisch ist. Der Film darf nicht spröde, rissig und brüchig werden.

Vereinigte Papierwerke
Schickedanz & Co.

Schoppershof 0156257
8500 Nürnberg

HP/5/ - B 71 D
26.03.1984

- 10 -

Desweiteren ist darauf zu achten, daß der Hot-melt-Film auf der Wäscheschutzfolie, die meistens aus Polyethylen oder Polypropylen besteht, gut haftet. Eine besonders gute Haftung ergibt sich bekanntermaßen auf Folien, die durch elektrische Corona-Entladung vorbehandelt sind. Man mißt in solchen Fällen eine Oberflächenspannung von größer als 38 mN/m. Auch Folien mit geprägter Oberfläche kommen in Betracht.

Die ausgewählte Hot-melt-Sorte muß eine lange offene Zeit haben, d. h. lange schmelzflüssig bleiben, damit eine hinreichende Penetration der unteren Schicht des Saugkörpers eintritt. Bewährt haben sich offene Zeiten von mehr als 5 Sekunden. Voraussetzung hierfür sind Rohstoffe hoher Dichte , geringer Wärmeleitfähigkeit und hoher spezifischer Wärme c, denn die Abkühlzeit t, also die offene Zeit, gehorcht der Proportionalität , wobei ist.

Soll, wie in der Zeichnung dargestellt, auch die Haftschmelzklebstoffschicht zum Aufbau des Stabilierungsblockes beitragen, so wird vorgeschlagen, einen Haftschmelzklebstoff einzusetzten, dessen Erweichungspunkt bei ca. 80 bis 120 °C, vorzugsweise bei 90 bis 110 °C, liegt. Die Applikationstemperatur soll 150 bis 220 °C, vorzugsweise 170 bis 200 °C betragen. Die Viskosität des Haftschmelzklebers bei 150 °C sollte bei ca. 3.000 bis 20.000 m Pas. ($D = 42$ s$^{-1}$) und bei 175 °C bei ca. 500 bis 10.000 m Pas. ($D = 63$ s$^{-1}$) liegen. Es werden bei 175 °C Werte von 1.000 bis 2.000 m Pas. bevorzugt. (Die angegebenen Viskositäten beziehen sich auf Messungen mit Rotationsviskosimetern im Bechersystem, wobei D das Geschwindigkeitsgefälle kennzeichnet.)

Vereinigte Papierwerke
Schickedanz & Co.

Schoopershefte 0156257
8500 Nürnberg

HP/5/ - B 71 D
26.03.1984

8

- 11 -

Die Zusammensetzung des Haftschmelzklebers ist in der einschlägigen Industrie üblich und bekannt; z. B. handelt es sich um Abmischungen von Polymeren auf Basis Polyacrylsäureester, Ethylenvinylacetat-Copolymere oder Styrol-Isopren/Butadien-Styrol-Block-Copolymeren mit klebrigmachenden Harzen, Weichmachern, Füllstoffen und Stabilisatoren.

Vereinigte Papierwerke
Schickedanz & Co.

Schoppershof **0156257**
8500 Nürnberg

HP/5/ - B 71 D
26.03.1984

Patentansprüche

1. Hygienischer Zellstoffartikel, wie Damenbinde oder
Slipeinlage mit einem Saugkörper, einer porösen
Vliesstoffabdeckung und einer Wäscheschutzfolie, bei
dem der Saugkörper wenigstens mit seiner unteren
Schicht mit der Wäscheschutzfolie verklebt ist und
die Wäscheschutzfolie oder der Zellstoffartikel auf
der Unterseite eine Haftschmelzkleberschicht aufweist,
dadurch gekennzeichnet,
daß die den Saugkörper (4) mit der Wäscheschutzfolie (9) verbindende Klebstoffschicht (12) aus einem
Heißschmelzklebstoff oder Dispersionsklebstoff besteht, der den Zellstoff-Artikel in Quer- und Längsrichtung versteift.

2. Hygienischer Zellstoffartikel nach Anspruch 1,
dadurch gekennzeichnet,
daß die Klebstoffschicht aus Heißschmelzklebstoff
besteht, der auf der Basis kurzkettiger Paraffinwachse hoher Kristallinität aufgebaut ist und der weitere
Anteile von mikrokristallinen Wachsen und/oder Elastomeren auf Basis Polyvinylacetat, Polyvinylpropionat, Äthylen-Vinylacetat-Copolymere, Polyacrylsäureester, Butadien-Styrol-Copolymere, Blockcopolymere auf
Basis Styrol-Isopren-Styrol oder Styrol-Butadien-Styrol, klebrigmachende Harze auf Basis Kolophonium und
seiner Derivate, sowie aliphatische, aromatische oder
alkylaromatische Kohlenwasserstoffharze enthält.

Vereinigte Papierwerke
Schickedanz & Co.

Schoppershofstr. 80
8500 Nürnberg

0156257

HP/5/ - B 71 D
26.03.1984

- 2 -

3. Hygienischer Zellstoffartikel nach Anspruch 1 oder 2,
dadurch gekennzeichnet,
daß die Klebstoffschicht (12) ein Flächengewicht von
30 bis 200 g/qm aufweist.

4. Hygienischer Zellstoffartikel nach Anspruch 3,
dadurch gekennzeichnet,
daß die Klebstoffschicht (12) ein Flächengewicht von
100 bis 150 g/qm aufweist.

5. Hygienischer Zellstoffartikel nach einem der vorausgehenden Ansprüche,
dadurch gekennzeichnet,
daß die Klebstoffschicht (12) wenigstens 2 mm, besser
5 bis 10 mm schmäler ist als die Breite des Saugkörpers.

6. Hygienischer Zellstoffartikel nach einem der vorausgehenden Ansprüche,
dadurch gekennzeichnet,
daß die Länge der Klebstoffschicht (12) das 0,5- bis
0,75-fache der Länge des Saugkörpers (4) beträgt und
zentral im Mittelbereich des Artikels angeordnet ist.

Vereinigte Papierwerke
Schickedanz & Co.

Schoppershofstr. 80
8500 Nürnberg

01 56 257

HP/5/ - B 71 D
26.03.1984

- 3 -

7. Hygienischer Zellstoffartikel nach einem der vorausgehenden Ansprüche, bei dem die poröse Vliesstoff-Abdeckung als Umhüllung ausgeführt ist, deren Ränder sich an der Unterseite des Zellstoff-Artikels überlappen und dort miteinander verklebt sind, dadurch gekennzeichnet, daß die Haftschmelzkleberschicht (11) die Vliesstoffumhüllung im Bereich der Unterseite wenigstens teilweise durchdringt (11')und mit der Unterseite der Wäscheschutzfolie (9) verbindet.

8. Hygienischer Zellstoffartikel nach Anspruch 7, dadurch gekennzeichnet, daß die Haftschmelzkleberschicht (11) die Vliesstoffumhüllung auch im Überlappungsbereich (7; 7') durchdringt und mit der Unterseite der Wäscheschutzfolie (9) verbindet.

0156257

Vereinigte Papierwerke                    Schoppershofstr. 80
Schickedanz & Co.                         8500 Nürnberg

                                          HP/5/ - B 71 D
                                          26.03.1984

- 12 -

Bezugszeichenliste

1        =    Damenbinde

2        =    Unterseite

3        =    Gebrauchsseite

4        =    Saugkörper

5        =    Vliesstoffschicht

6        =    Rand

7; 7'    =    Überlappende Teile von 5

8        =    Klebstoffnaht

9        =    Wäscheschutzfolie

10       =    Ränder von 9

11,11'   =    Haftschmelzkleberschicht

12       =    Klebstoffschicht

0156257